# FASCICULE DE BREVET EUROPEEN

(11) **EP 2 789 290 B1**
(45) Date de publication et mention de la délivrance du brevet: **06.11.2019**
(21) Numéro de dépôt: 14164078.9
(22) Date de dépôt: 09.04.2014
(51) Int. Cl.: A61B 1/227, A61B 1/233, A61B 1/24, H04M 1/21, A45C 11/38

(54) **Dispositif pour l'acquisition d'images d'une zone d'un corps humain ou animal**
Vorrichtung zur Bilderfassung einer Zone des Körpers eines Menschen oder Tieres
Device for acquiring images of a region of a human or animal body

(30) Priorité: 09.04.2013 FR 1353176
(43) Date de publication de la demande: 15.10.2014
(73) Titulaire: Schmoll, Laurent, 67000 Strasbourg (FR)
(72) Inventeur: Schmoll, Laurent, 67000 Strasbourg (FR)
(74) Mandataire: IP Trust

(56) Documents cités:
- WO-A2-2012/058641
- US-A1- 2012 275 025

## Description

### DOMAINE TECHNIQUE DE L'INVENTION.

L'invention concerne un dispositif pour l'acquisition d'images d'une cavité ou d'un conduit ou d'une zone d'un corps humain ou animal.

Le dispositif d'acquisition selon l'invention est destiné notamment, mais non exclusivement, à l'examen de cavités buccales, de conduits auditifs, des fosses nasales, ou de la peau.

### ETAT DE LA TECHNIQUE

De manière traditionnelle, les étapes de recueil des données médicales auprès d'un patient, l'établissement d'un diagnostic et le cas échéant l'établissement d'un traitement thérapeutique sont réalisés au cours d'une consultation du patient par le médecin.

Cependant, le développement des technologies dans le domaine de l'information et de la communication et en particulier l'émergence d'Internet dans le domaine de la santé observés ces dernières années favorise le recours à des soins à distance. Ce développement donne de ce fait une nouvelle dimension à l'exercice médical qui voit apparaitre la télémédecine et ses applications comme par exemple la téléconsultation. Le document WO-A-2012/058641 décrit un dispositif pour l'acquisition d'image selon le préambule de la revendication 1.

Dans le cas de troubles touchant notamment la gorge, les oreilles ou le nez, il s'avère nécessaire en général d'examiner les cavités buccales, les conduits auditifs externes ou des tympans ou les fosses nasales pour établir un diagnostic. Or, les instruments traditionnels utilisés pour l'inspection de ces cavités ou conduits, et en particulier l'otoscope utilisé pour l'inspection des conduits auditifs et le spéculum pour les fosses nasales, s'avèrent inadaptés dans le cadre de téléconsultations. Il en est de même pour les affections touchant la peau.

L'invention vise à remédier à ce problème en proposant un dispositif d'acquisition d'images de zones d'un corps humain ou animal permettant au patient, de manière simple et rapide, de réaliser des photographies transférables par exemple à un médecin aux fins d'être exploitées dans le cadre d'une téléconsultation.

### OBJET DE L'INVENTION

A cet effet, et selon un premier aspect, l'invention propose un dispositif pour l'acquisition d'images d'une zone d'un corps humain ou animal en vue d'un examen médical à distance selon la revendication 1.

En équipant un téléphone cellulaire, ou tout autre appareil de prise d'images, d'un support pourvu d'une tête de visée telle que définie ci-dessus, la capture d'images en particulier de cavités ou de conduits mais également de tout zone telle que la peau, et le transfert de ces images à des médecins, plateformes médicales ou autres en vue d'une consultation à distance est rendue possible.

Avantageusement, la tête de visée présente un profil évasé. Selon l'organe visé, la tête de visée sera évasée en direction de l'organe ou dans une direction opposée.

Avantageusement, la tête de visée est montée de manière amovible sur le support. Cela permet de changer les têtes de visée, et de choisir une tête de visée présentant une taille adaptée à la cavité ou au conduit à examiner et/ou au patient concerné (enfant ou adulte).

Selon un mode de réalisation avantageux, le support comporte une embase de fixation de la tête de visée.

Avantageusement, le corps tubulaire de la tête de visée comporte un ergot apte à coopérer avec une fente ménagée sur l'embase de fixation ou tout système de type baïonnette.

Avantageusement, l'embase de fixation présente une forme complémentaire à celle de la tête de visée de manière à permettre un emboitement de la tête de visée avec ladite embase.

Selon un autre mode de réalisation, la tête de visée et le support sont formés d'un seul tenant.

Selon un autre mode de réalisation, la tête de visée est jetable.

Il peut être prévu également que la tête de visée comporte un système optique permettant d'agrandir l'image de la zone examiné(e).

Selon un mode de réalisation particulier, la tête de visée comprend une partie optique formée par le corps de la tête de visée et par au moins une lentille située à l'intérieur du canal.

Avantageusement, la tête de visée comporte une partie endoscopique disposée dans le prolongement axial de la partie optique. Il peut être prévu alors que la partie endoscopique soit solidaire de la partie optique ou montée de manière amovible sur celle-ci.

Selon une autre variante de réalisation, la tête de visée comporte à son extrémité opposée à celle fixée au support, une bague de couplage permettant le couplage d'un endoscope ou d'une partie endoscopique à la tête de visée.

Les moyens lumineux ainsi arrangés avec les moyens de guidage permettent d'améliorer la visée de la zone examinée. Selon une configuration particulière, lesdits moyens lumineux sont portés par la tête de visée. Une batterie fixée sur la coque permet d'éclairer la zone à photographier avant la prise du cliché.

Selon un mode de réalisation préféré, le support est une coque de protection pour téléphone cellulaire.

L'invention concerne également un kit pour l'acquisition d'images d'une zone d'un corps humain ou animal en vue d'un examen médical comprenant au moins un support pour recevoir un téléphone cellulaire et une pluralité de têtes de visée de la zone à examiner, le support et la tête de visée définissant un dispositif d'acquisition selon l'une quelconque des revendications précédentes.

### BREVE DESCRIPTION DES FIGURES

D'autres objets et avantages de l'invention apparaîtront au cours de la description qui suit, faite en référence aux dessins annexés, dans lesquels :
- la figure 1 représente une vue en perspective de face d'un dispositif d'acquisition d'images d'une zone d'un corps humain qui ne fait pas partie de l'invention ;
- la figure 2 représente une vue de côté du dispositif d'acquisition de la figure 1 ;
- la figure 3 représente une vue latérale d'un support selon un exemple de réalisation ;
- la figure 4 représente une vue en perspective d'une tête de visée destinée à être fixée sur le support de la figure 3 ;
- les figures 5 et 6 représentent respectivement une vue latérale et une vue de face du support selon un autre exemple de réalisation ;
- la figure 7 représente une vue de détail d'un système optique en appui sur le dispositif d'acquisition de la figure 5 ;
- la figure 8 représente une vue en perspective d'une tête de visée pour l'examen de cavités buccales ;
- la figure 9 représente une vue en perspective d'une tête de visée pour l'examen de conduits auditifs ;
- la figure 10 représente une vue en perspective d'une tête de visée pour l'examen de fosses nasales ;
- la figure 11 représente une vue en perspective d'une tête de visée pour l'examen d'une zone de peau ;
- les figures 12a et 12b représentent une vue de face et de côté d'un dispositif d'acquisition d'images ;
- les figures 13 et 14 représentent une vue respectivement de côté et de dessus d'un dispositif d'acquisition d'images l'invention ;
- la figure 15 représente une variante de réalisation de la tête de visée illustrée sur les figures 13 et 14 ;
- la figure 16 représente une autre variante de réalisation de la tête de visée illustrée sur les figures 13 et 14 ;
- la figure 17 représente une vue de côté d'un dispositif d'acquisition d'images selon un autre mode de réalisation de l'invention ;
- la figure 18 représente une vue de la tête de visée du dispositif de la figure 17 ;
- la figure 19 représente une variante de réalisation du dispositif d'acquisition d'images de la figure 17.

### DESCRIPTION DETAILLEE DES FIGURES

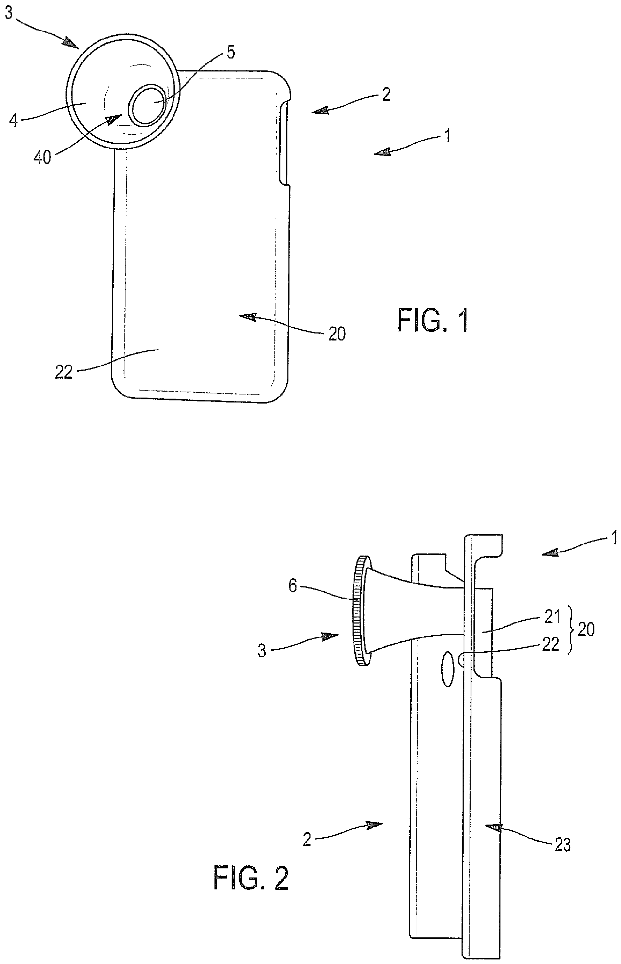

En relation avec les figures 1 et 2, il est décrit un dispositif d'acquisition d'images d'une cavité du corps humain telle qu'une bouche permettant, lorsqu'il est associé à un appareil de prise d'images, tel qu'un téléphone cellulaire, une tablette ou autres, de prendre des photographies ou des films.

Pour ce faire, le dispositif d'acquisition 1 comprend un support 2 destiné à être monté sur l'appareil de prise d'images et une tête de visée 3 portée par le support 2. Dans l'exemple décrit, l'appareil de prise d'image est un téléphone intelligent, par la suite désigné sous le terme de smartphone. Le support du dispositif d'acquisition 1 se présente sous la forme d'une coque de protection d'un smartphone. Il est bien entendu évident que le dispositif d'acquisition 1 peut être mis en œuvre avec d'autre type d'appareil permettant la prise d'images et que dans ce cas, le support pourra prendre d'autres formes que celle décrite dans la présente demande.

Ainsi, dans le mode de réalisation illustré, le support 2 se présente sous la forme d'une coque de protection rigide ou semi-rigide présentant une forme sensiblement complémentaire à celle d'un smartphone. Le support 2 présente ainsi une paroi principale 20 présentant une face interne 21 destinée à être plaquée contre la face arrière du smartphone et une face externe 22, la paroi principale 20 étant prolongée par une bordure latérale 23 agencée pour se plaquer contre les faces latérales du smartphone. Le support 2 comporte en outre un orifice de passage disposé de façon à faire face à la lentille de caméra du téléphone cellulaire lorsque ce dernier est reçu par le support et ainsi permettre le passage des images vers le smartphone.

On entend par « face externe », la face visible une fois le support 2 monté sur l'appareil de prise d'image, et a contrario par « face interne », la face cachée une fois le support 2 monté sur l'appareil de prise d'image.

La tête de visée 3, portée par la face externe 22 du support 2, présente un corps tubulaire 4 délimité par des ouvertures d'extrémités 5, 6.

Avantageusement, la tête de visée 3 présente un profil tronconique. Dans le mode de réalisation illustré, la tête de visée 3 est évasée dans la direction opposée à la face externe 12 du support 2 (forme en trompette). Le dispositif d'acquisition 1 est ainsi adapté à l'examen d'une cavité buccale.

Dans le mode de réalisation, la tête de visée 3 est représentée en partie supérieure, à gauche de la coque de protection de sorte que lorsque le support 2 est monté sur le smartphone, le canal de visée 40 de la tête de visée 3 délimité par le corps de tête de visée 4 et les ouvertures d'extrémité 5, 6 est placé dans l'alignement de l'orifice du support 2. Il est bien entendu évident que la position de la tête de visée 3 sur la coque de protection n'est pas limitée à cet emplacement, ce dernier étant fonction de l'emplacement de la lentille de caméra du dispositif de prise d'image sur lequel le support 2 est destiné à être monté.

Avantageusement, la tête de visée 3 est fixée de manière amovible sur le support 2. La tête de visée 3 peut être par exemple pourvue au niveau de l'ouverture d'extrémité 5 d'un filetage apte à coopérer avec un filetage interne ménagé au niveau de l'orifice traversant prévu dans la paroi principale 20 du support 2 pour permettre à le lentille de caméra du téléphone cellulaire de recevoir l'image de la zone examinée. Selon une variante de réalisation, les moyens de fixation prévus sur la tête de visée 3 et le support 2 peuvent être du type baïonnette. Le caractère amovible de la tête de visée a pour avantage de permettre de changer de tête de visée lorsque celle-ci est défectueuse, d'adapter la tête de visée 3 à la cavité, au conduit ou plus généralement à la zone à examiner. Ainsi, une même coque de protection pourra servir par exemple à la fois de support de tête pour la visée d'une cavité buccale, de support de tête pour la visée d'un conduit auditif et de support de tête pour la visée d'une zone de peau.

Afin d'agrandir l'image de la cavité ou du conduit examiné, la tête de visée 3 comporte avantageusement un système optique, du type loupe.

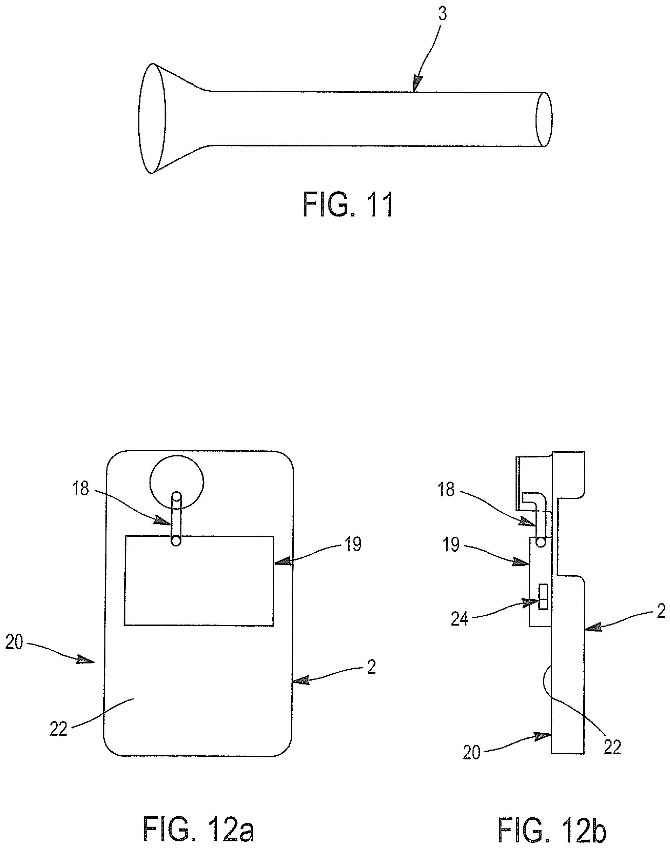

De même, afin de faciliter l'examen de la cavité et d'améliorer les photographies prises par l'appareil de prise d'image, le dispositif d'acquisition comporte des moyens lumineux pour éclairer la cavité, ces moyens étant intégrés de préférence dans la tête de visée 3. Un exemple de réalisation est illustré sur les figures 12a et 12b. Dans ce mode de réalisation, les moyens lumineux sont formés par une fibre optique 18 alimentée par une batterie 19. La batterie 19 est ménagée sur le support 2 (dans l'exemple illustré, la batterie est portée sur la face externe 22 de la paroi principale 20). La batterie est avantageusement pourvue d'un interrupteur de batterie 24.

Dans le mode de réalisation illustré, la tête de visée 3 présente un corps en plastique pourvu d'un revêtement métallique. Il est bien entendu évident que le corps de la tête de visée 3 peut être réalisé en tout type de matériau et qu'il peut être revêtu ou non d'une couche de protection ou d'une couche à effet esthétique sans sortir du cadre de l'invention.

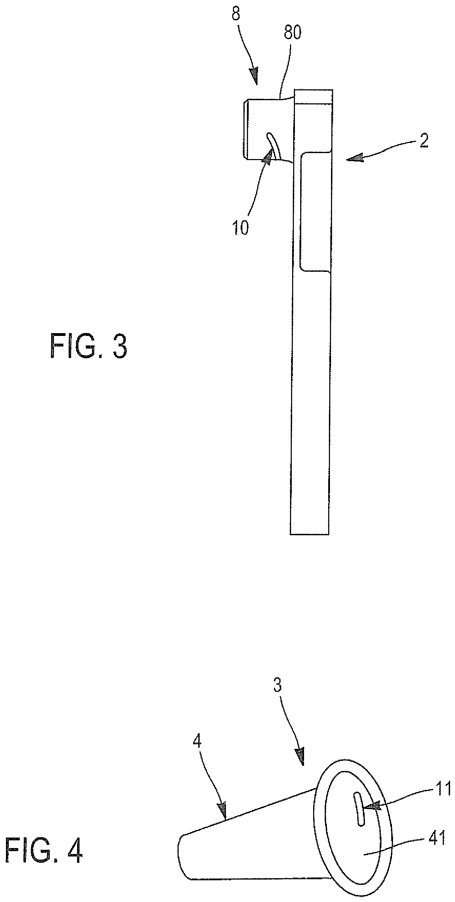

Les figures 3 et 4 illustrent le dispositif d'acquisition selon une variante de réalisation. Dans cette variante de réalisation, le support 2 comporte une embase de fixation 8 fixe pour la fixation de la tête de visée 3. Dans l'exemple illustré, l'embase de fixation 8 présente un corps tubulaire 9 de forme sensiblement complémentaire à la forme de la tête de visée 3 de manière à permettre un emboitement de la tête de visée 3 avec ladite embase. L'embase de fixation 8 est formée d'un seul tenant avec le support 2.

Avantageusement, le dispositif 1 comporte des moyens de fixation de la tête de visée 2 sur l'embase de fixation 8. Dans le mode de réalisation illustré, les moyens de fixation comprennent une fente circonférentielle 10 ménagée sur la face extérieure 80 du corps de l'embase de fixation 8 et un ergot interne 11, de forme complémentaire à la fente circonférentielle, ménagé sur la face interne 41 du corps de tête 4, au niveau de l'extrémité d'ouverture 5. Ainsi, la fixation de la tête de visée 3 sur le support 2 est réalisée par emboitement de la tête de visée sur l'embase de fixation 8 jusqu'au clipsage de l'ergot interne 11 de la tête de visée 3 dans la fente 10 de l'embase de fixation 8. Avantageusement, la fente 10 ménagée sur l'embase de fixation 8 est agencée pour former une rampe de manière à permettre le retrait de la tête de visée 2 par un mouvement de rotation sur l'embase suivi d'un mouvement de coulissement en direction opposée au support 2.

Il s'agit bien entendu d'un exemple de fixation étant entendu que d'autres types de fixation, comme par exemple une fixation par vissage, peuvent être mis en œuvre sans sortir du cadre de l'invention.

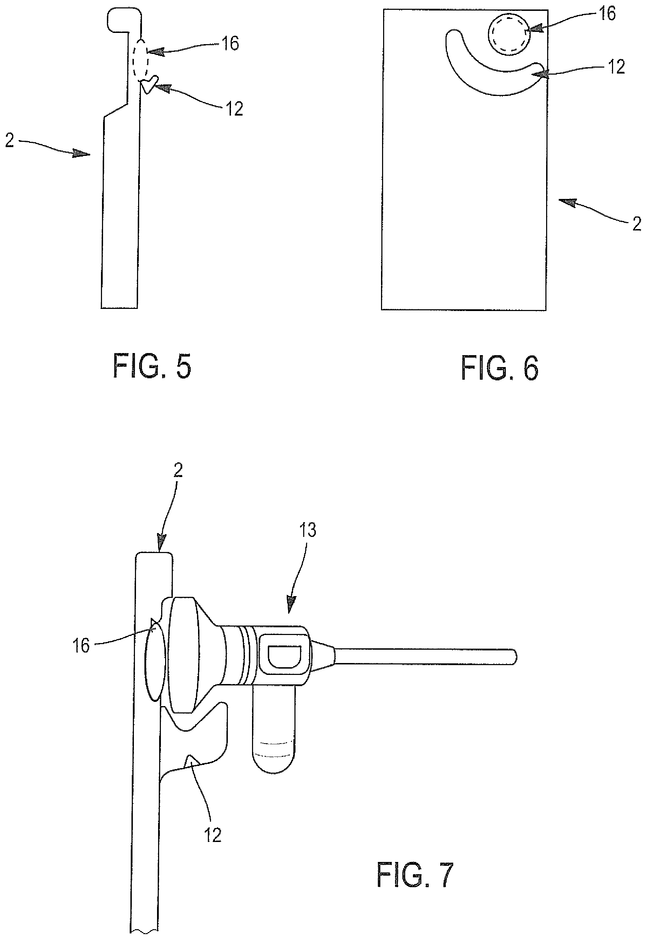

Les figures 5 et 6 illustrent un dispositif d'acquisition selon un autre mode de réalisation. Dans ce mode de réalisation, le support 2 présente, sur sa face extérieure, un ergot d'appui 12 apte à soutenir un système optique. Dans le mode de réalisation illustré, l'ergot d'appui 12 présente un profil en demi-lune. Il est agencé sous l'orifice traversant permettant à la lentille de caméra du téléphone cellulaire de recevoir les images de la zone examinée et conformé pour soutenir une fibre optique pour oreille 13 comme illustré sur la figure 7. Avantageusement, le support 2 comporte une lentille intégrée 16 ménagée au travers de l'orifice.

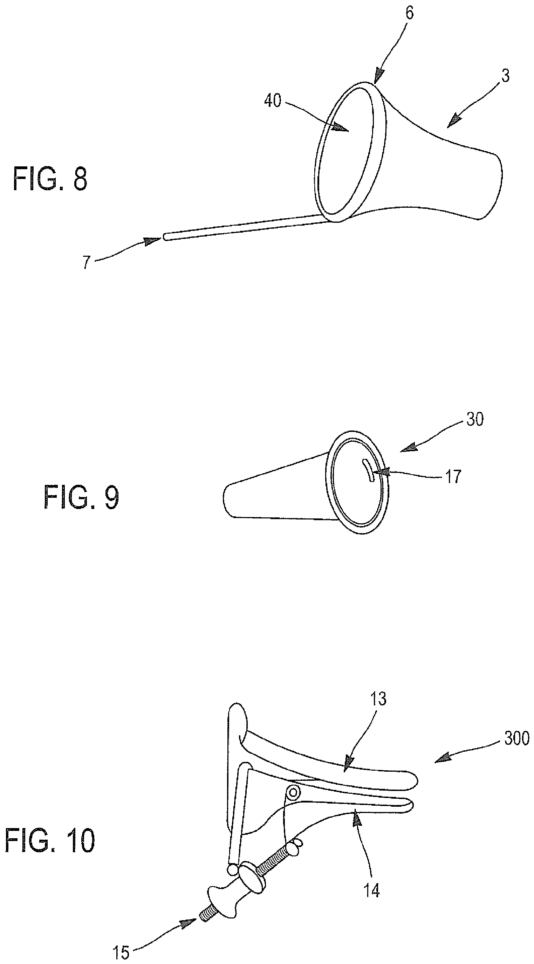

Afin de faciliter l'examen de la cavité buccale, il peut prévu, selon une variante de réalisation avantageuse, une tête de visée 3 pourvue d'une lame plane 7 pour abaisser la langue durant l'examen de la cavité buccale (figure 8). La lame 7 est agencée avec la tête de visée 2 pour s'étendre en partie avant de la tête, dans un plan passant par la collerette 60 délimitant l'ouverture d'extrémité 6 parallèle à l'axe du canal de visée 40. On définit l'avant de la tête de visée 2 par rapport à la zone du corps humain ou animal. Avantageusement, la lame 7 et la tête de visée 2 sont formées d'une seul tenant.

Dans l'exemple précédemment décrit, la tête de visée 3 présente un profil évasée dans la direction opposée à la face externe 22 du support 2 (forme en trompette). Le dispositif d'acquisition 1 est ainsi adapté à l'examen d'une cavité buccale. La tête de visée 3 n'est cependant pas limitée à une telle forme, cette dernière pouvant être adaptée selon la zone à examiner.

Ainsi, dans le cas par exemple de l'examen d'un conduit auditif, la tête de visée présentera une forme allongée se rétrécissant en direction opposée à la face externe 22 du support 2. Une telle tête de visée est représentée sur la figure 9. Avantageusement, il est prévu d'équiper la tête de visée 2 au niveau de l'extrémité de raccordement (extrémité 5) avec le support d'une lentille 17 afin d'agrandir les images du conduit auditif visualisées (figure 9).

La figure 10 illustre une tête de visée 3 mise en œuvre pour l'inspection des fosses nasales. Plus particulièrement, la tête de visée 3 comporte deux bras 13, 14, l'un des bras (dans l'exemple illustré le bras inférieur 14) étant mobile par rapport à l'autre bras (bras supérieur 13) de sorte à agrandir ou diminuer le canal de visée. Selon un mode de réalisation particulier, le mouvement du bras inférieur 14 peut être actionné à l'aide d'un système de vis 15, comme illustré sur la figure 10, ou à l'aide d'un système à ressort.

La figure 11 illustre une tête de visée 3 pour l'examen de la peau (on parle alors de tête de visée ou d'embout cutané). Selon cette application, la tête de visée 3 présente un corps tubulaire allongé de section sensiblement constante présentant, en extrémité avant, une zone élargie en forme d'entonnoir. Avantageusement, la tête de visée 3 est réalisée en matière plastique transparente afin de faciliter le positionnement du dispositif sur la zone à examiner.

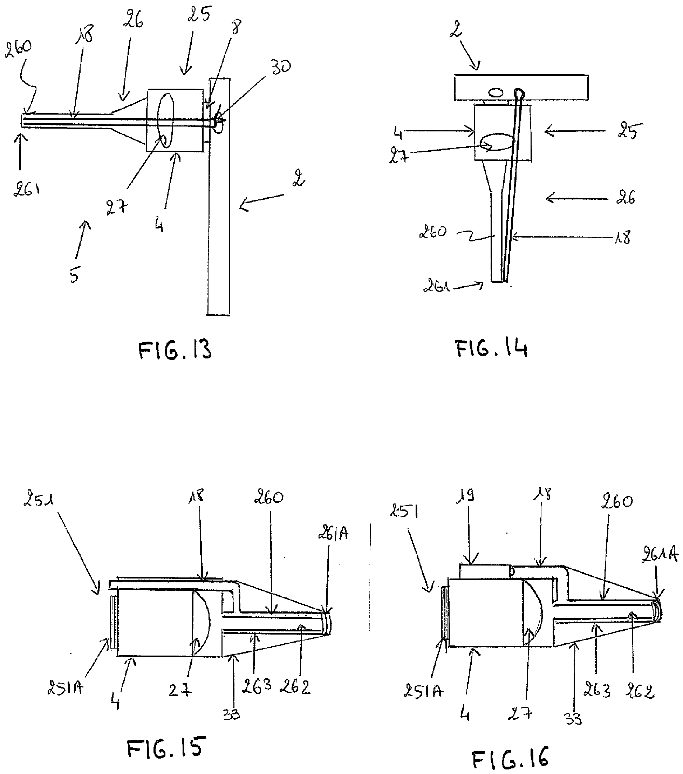

Les figures 13 et 14 illustrent un autre mode de réalisation du dispositif d'acquisition d'images 100. Dans ce mode de réalisation, le dispositif d'acquisition 100 comporte une partie optique 25 et une partie endoscopique 26 disposée dans le prolongement axial de la partie optique 25.

Par partie endoscopique, on entend une partie agencée pour assurer les fonctions d'un endoscope. La partie endoscopique 26 est formée d'un tube 260 similaire à celui d'un endoscope de façon à conduire l'image réelle au plan image de l'objectif.

La partie optique 25, délimitée par le corps tubulaire 4 de la tête de visée 3, comprend une lentille 27 située à l'intérieur du canal 40 du corps 4. Dans le mode de réalisation illustré, la partie optique 25 comprend une seule lentille 27. Il est bien entendu évident que l'invention ne se limite pas à cette configuration et que la partie optique peut comprendre plusieurs lentilles.

Avantageusement, le dispositif d'acquisition 100 comporte avantageusement des moyens lumineux pour éclairer la cavité. Dans le mode de réalisation illustré sur les figures 13 et 14, les moyens lumineux comportent une source indépendante lumineuse 30, du type LED, ménagée au niveau du support 2 et des moyens de guidage 18, du type fibre optique, pour guider le flux de lumière en provenance de la source lumineuse jusqu'à l'extrémité libre 261 (extrémité distale) de la partie endoscopique 26. Dans le mode de réalisation illustré, la fibre optique est agencée pour présenter une trajectoire rectiligne depuis les moyens lumineux vers l'extrémité distale de la partie endoscopique pour présenter une ouverture de sortie adjacente à l'extrémité distale 261 du tube endoscopique 260. Il est bien entendu évident que l'invention n'est pas limitée à cette configuration et qu'il peut être prévu une fibre optique (ou tout autre moyens de guidage) agencée pour longer en tout ou partie les surfaces extérieures des parties constitutives des parties optiques et endoscopiques comme illustré sur la figure 15 ou pour traverser le corps 4 de la tête de visée 3.

Selon une configuration particulière illustrée sur la figure 15, le tube endoscopique 260 est formée d'une double paroi de sorte à définir deux conduits coaxiaux 262, 263, le conduit externe 263 étant raccordé à la source lumineuse indépendante 30 par l'intermédiaire d'une fibre optique. Le conduit externe 263 permet ainsi de transporter le flux lumineux depuis la source lumineuse 30 jusqu'au point de visée, tandis que le conduit interne 262 permet de conduire l'image réelle au plan image de l'objectif.

Dans les modes de réalisation précédemment décrits, les moyens lumineux sont portés par le support 2. Les moyens lumineux peuvent être prévus également au niveau de la partie optique 25. La figure 16 illustre un exemple de réalisation où la fibre optique 18 est alimentée par une batterie 19 séparée ménagée sur le corps 4 de la partie optique 25, la batterie permettant d'envoyer un flux lumineux au sein de la fibre optique 18.

Il peut être également prévu, selon un mode de réalisation ne faisant pas partie de l'invention, que les moyens lumineux mis en œuvres ne soient pas un élément constitutif du dispositif d'acquisition 100 mais celui de l'appareil de prise d'images comme par exemple comme la LED du flash de ce dernier. Selon cette configuration, les moyens de guidage s'étendront alors depuis un orifice ménagé dans le support de manière à être disposé en vis-à-vis de la LED de l'appareil de prise d'image et s'étendront à l'intérieur ou l'extérieur de la partie optique 25 jusqu'à l'extrémité distale du tube endoscopique 260 ou jusqu'au conduit externe 263 du tube selon la configuration de ce dernier.

Avantageusement, l'extrémité distale 261 du tube endoscopique présente un filetage 261A afin de permettre le positionnement d'une pièce de protection 33 illustrée sur les figures 15 et 16. Avantageusement, la pièce de protection 33, de préférence en plastique et à usage unique, présente une forme conique Cette pièce de protection 33 permet d'une part d'apporter une protection au tube 260, réalisé en général en métal, de la partie endoscopique 26 et d'autre part de préserver l'hygiène de la partie endoscopique, celui-ci n'étant pas en contact avec les orifices examinés.

Avantageusement, la partie optique 25 est montée de manière amovible sur le support 2. Pour ce faire, le corps 4 de la partie optique 25 de la tête de visée 3 présente une extrémité libre 251 pourvue d'un filetage 251A apte à coopérer avec un filetage complémentaire ménagé au niveau du support 2, et de préférence sur une embase de fixation 8 du support 2 prévue à cet effet. Il est bien entendu évident que l'invention ne se limite pas à cette configuration et qu'il peut être prévu d'autres moyens de fixation permettant une fixation amovible de la partie optique 25 sur le support 2 sans sortir du cadre de l'invention (système de baïonnette, etc.).

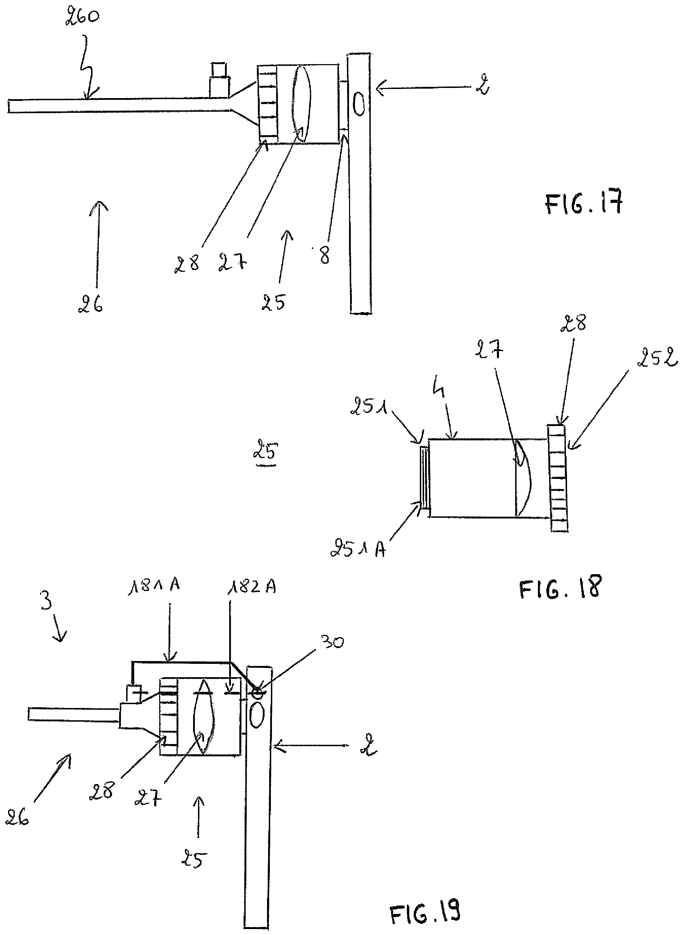

Dans le mode de réalisation décrit, la partie endoscopique 26 est formée d'un seul tenant avec la partie optique 25. L'ensemble forme ainsi une tête de visée monobloc. Selon une variante de réalisation illustrée sur les figures 17 à 19, la partie endoscopique 26 est montée de manière amovible sur la partie optique 25, elle-même configurée pour être montée de manière amovible sur le support 2. Dans le mode de réalisation illustré, le couplage de la partie endoscopique sur la partie optique 25 est réalisé au moyen d'une bague de couplage 28. Avantageusement, la bague de couplage 28 est fixée de manière non amovible à l'extrémité 252 opposée à l'extrémité 251 destinée à être fixée au support 2. Il peut bien entendu être prévu une bague fixée de manière amovible à ladite extrémité 252. L'avantage de ce mode de réalisation est de permettre d'adapter le type (endoscope ou fibroscope) et la taille de partie endoscopique en fonction de la cavité à examiner et d'ainsi offrir un dispositif d'acquisition modulaire.

Dans ce type de configuration, les moyens de guidage de la lumière pourront être extérieure à la partie optique (ligne 18A de la figure 19) ou interne (ligne 18B de la figure 19).

Dans les modes de réalisation décrits, le tube endoscopique 260 est rigide. Il est bien entendu évident que l'invention ne se limite pas à ce type de tube et qu'il peut être prévu un tube endoscopique flexible.

Dans les modes de réalisation précédemment décrits, la tête de visée 3 est montée de manière amovible sur le support 2. Il est bien entendu évident que l'invention ne se limite pas à cette configuration et qu'il peut être prévu un dispositif ayant une tête de visée formée d'un seul tenant avec le support 2 (figure 3).

L'avantage d'un dispositif d'acquisition selon l'invention est de permettre de prendre des photographies ou des films d'une cavité ou d'un conduit du corps humain par le biais de son smartphone lorsque la coque de protection est placée sur celui-ci, d'enregistrer les photographies ou films pris par le smartphone et de transférer ces données à son médecin, à un centre médical ou une plateforme Internet par les moyens classiques de transmission d'information tels que SMS, MMS, emails, et similaires, et permettre ainsi au médecin ou au personnel médical d'établir des diagnostics à distance à partir des photographies ou films reçus.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Dispositif pour l'acquisition d'images (1, 100) d'une zone d'un corps humain ou animal en vue d'un examen médical à distance comprenant un support (2) pour recevoir un téléphone cellulaire, le support (2) comprenant un orifice de passage arrangé pour faire face à la lentille de caméra du téléphone cellulaire lorsque ce dernier est reçu par le support (2), et une tête de visée (3) de la zone à examiner présentant un corps (4) tubulaire délimitant un canal (40) traversant s'étendant longitudinalement, ladite tête de visée étant agencée avec le support (2) de façon à ce que le canal (40) soit disposé dans l'alignement de l'orifice du support (2), la tête de visée (3) comprenant une partie optique (25) formée par le corps (4) de la tête de visée et par au moins une lentille (27) située à l'intérieur du canal (40) et une partie endoscopique (26) disposée dans le prolongement axial de la partie optique (25), le dispositif comprenant en outre des moyens lumineux et des moyens de guidage du flux de lumière en provenance des moyens lumineux, **caractérisé en ce que** les moyens lumineux sont ménagés sur le support ou intégrés dans la tête de visée, lesquels moyens lumineux comportent, lorsqu'ils sont ménagés sur le support, une source indépendente lumineuse (30) du type LED, lesdits moyens de guidage (18) du flux de lumière s'étendant depuis les moyens lumineux jusqu'à l'extrémité distale de la partie endoscopique (26).

2. Dispositif pour l'acquisition d'images (1, 100) d'une zone selon la revendication 1, **caractérisé en ce que** la tête de visée (3) présente un profil tronconique.

3. Dispositif pour l'acquisition d'images (1, 100) d'une zone selon la revendication 1 ou la revendication 2, **caractérisé en ce que** la tête de visée (3) est montée de manière amovible sur le support (2).

4. Dispositif pour l'acquisition d'images (1, 100) d'une zone selon la revendication 4, **caractérisé en ce que** le support (2) comporte une embase de fixation (8) de la tête de visée, ladite embase présentant une forme complémentaire à celle de la tête de visée (3) de manière à permettre un emboitement de la tête de visée avec ladite embase.

5. Dispositif pour l'acquisition d'images d'une zone selon la revendication 1 ou la revendication 2, **caractérisé en ce que** la tête de visée (3) et le support (2) sont formés d'un seul tenant.

6. Dispositif pour l'acquisition d'images (1, 100) d'une zone selon l'une quelconque des revendications 1 à 5, **caractérisé en ce que** le dispositif (1) comporte un système optique pour agrandir l'image de la cavité ou du conduit examiné.

7. Dispositif pour l'acquisition d'images (1, 100) d'une zone selon l'une quelconque des revendications 1 à 6, **caractérisé en ce que** la partie endoscopique (26) est solidaire de la partie optique (25).

8. Dispositif pour l'acquisition d'images (1, 100) d'une zone selon l'une quelconque des revendications 1 à 6, **caractérisé en ce que** la partie endoscopique (26) est montée de manière amovible sur la partie optique (25).

9. Dispositif pour l'acquisition d'images (1, 100) d'une zone selon l'une quelconque des revendications 1 à 8, **caractérisé en ce que** la tête de visée (3) comporte à son extrémité (252) opposée à celle fixée au support (2), une bague de couplage (28) permettant le couplage d'un endoscope ou -de la partie endoscopique (26) à la tête de visée (3).

10. Dispositif pour l'acquisition d'images (1, 100) d'une zone selon l'une quelconque des revendications 1 à 9, **caractérisé en ce que** le support est une coque de protection pour téléphone cellulaire.

11. Kit pour l'acquisition d'images d'une zone d'un corps humain ou animal en vue d'un examen médical comprenant au moins un support (2) pour recevoir un téléphone cellulaire et une pluralité de têtes de visée (3) de la zone à examiner, le support (2) et la tête de visée (3) définissant un dispositif d'acquisition (1, 100) selon l'une quelconque des revendications précédentes.

## Patentansprüche

1. Vorrichtung zur Bilderfassung (1, 100) einer Zone des Körpers eines Menschen oder Tieres zur Ferndiagnose, die eine Halterung (2) zur Aufnahme eines Mobiltelefons umfasst, wobei die Halterung (2) eine Durchgangsöffnung umfasst, die so angeordnet ist, dass sie sich gegenüber der Kameralinse des Mobiltelefons befindet, wenn dieses von der Halterung (2) aufgenommen wird, und einen Visierkopf (3) für den zu untersuchenden Bereich mit einem rohrförmigen Körper (4), der einen sich in Längsrichtung erstreckenden durchgängigen Kanal abgrenzt, wobei der Visierkopf mit der Halterung (2) so angeordnet ist, dass der Kanal (40) auf die Öffnung der Halterung (2) ausgerichtet angeordnet ist, wobei der Visierkopf (3) einen optischen Teil (25) umfasst, der gebildet wird durch den Körper (4) des Visierkopfs und durch zumindest eine im Innern des Kanals (40) befindliche Linse (27), und einen in der axialen Verlängerung des optischen Teils (25) angeordneten endoskopischen Teil (26), wobei die Vorrichtung des Weiteren Leuchtmittel und Mittel zum Führen des von den Leuchtmitteln kommenden Lichtstroms umfasst, **dadurch gekennzeichnet, dass** die Leuchtmittel auf der Halterung angebracht oder in den Visierkopf integriert sind, wobei die Leuchtmittel, wenn sie auf der Halterung angebracht sind, eine unabhängige Leuchtquelle (30) vom Typ LED umfassen, wobei sich die Mittel zum Führen (18) des Lichtstroms von den Leuchtmitteln aus bis zum distalen Ende des endoskopischen Teils (26) erstrecken.

2. Vorrichtung zur Bilderfassung (1, 100) einer Zone nach Anspruch 1, **dadurch gekennzeichnet, dass** der Visierkopf (3) ein kegelstumpfförmiges Profil aufweist.

3. Vorrichtung zur Bilderfassung (1, 100) einer Zone nach Anspruch 1 oder Anspruch 2, **dadurch gekennzeichnet, dass** der Visierkopf (3) abnehmbar auf der Halterung (2) montiert ist.

4. Vorrichtung zur Bilderfassung (1, 100) einer Zone nach Anspruch 4, **dadurch gekennzeichnet, dass** die Halterung (2) einen Sockel zum Befestigen (8) des Visierkopfes umfasst, wobei der Sockel eine die Form des Visierkopfes (3) ergänzende Form aufweist, so dass der Visierkopf in den Sockel eingreift.

5. Vorrichtung zur Bilderfassung (1, 100) einer Zone nach Anspruch 1 oder Anspruch 2, **dadurch gekennzeichnet, dass** der Visierkopf (3) und die Halterung (2) aus einem Stück ausgebildet sind.

6. Vorrichtung zur Bilderfassung (1, 100) einer Zone nach einem der Ansprüche 1 bis 5, **dadurch gekennzeichnet, dass** die Vorrichtung (1) ein optisches System zur Vergrößerung des Bildes der untersuchten Mundhöhle oder des untersuchten Gehörgangs umfasst.

7. Vorrichtung zur Bilderfassung (1, 100) einer Zone nach einem der Ansprüche 1 bis 6, **dadurch gekennzeichnet, dass** der endoskopische Teil (26) fest mit dem optischen Teil (25) verbunden ist.

8. Vorrichtung zur Bilderfassung (1, 100) einer Zone nach einem der Ansprüche 1 bis 6, **dadurch gekennzeichnet, dass** der endoskopische Teil (26) abnehmbar auf den optischen Teil (25) montiert ist.

9. Vorrichtung zur Bilderfassung (1, 100) einer Zone nach einem der Ansprüche 1 bis 8, **dadurch gekennzeichnet, dass** der Visierkopf (3) an seinem Ende (252) gegenüber dem an der Halterung (2) befestigten Ende einen Kopplungsring (28) umfasst, der das Ankoppeln eines Endoskops oder des endoskopischen Teils (26) am Visierkopf (3) ermöglicht.

10. Vorrichtung zur Bilderfassung (1, 100) einer Zone nach einem der Ansprüche 1 bis 9, **dadurch gekennzeichnet, dass** die Halterung eine Schutzschale für ein Mobiltelefon ist.

11. Set zur Bilderfassung einer Zone des Körpers eines Menschen oder Tieres zur Ferndiagnose, welches mindestens eine Halterung (2) zur Aufnahme eines Mobiltelefons und eine Vielzahl von Visierköpfen (3) der zu untersuchenden Zone umfasst, wobei die Halterung (2) und der Visierkopf (3) eine Erfassungsvorrichtung (1, 100) nach einem der vorhergehenden Ansprüche bilden.

## Claims

1. A device for acquiring images (1, 100) of an area of a human or animal body for remote medical examination comprising a support (2) for receiving a cellular telephone, the support (2) comprising a passage opening arranged to face the camera lens of the cellular telephone when the latter is received by the support (2), and a sighting head (3) of the area to be examined having a tubular body (4) defining a longitudinally extending through-channel (40), said sighting head being arranged with the support (2) so that the channel (40) is arranged in alignment with the opening of the support (2), the sighting head (3) comprising an optical part (25) formed by the body (4) of the sighting head and by at least one lens (27) located inside the channel (40) and an endoscopic part (26) arranged in the axial extension of the optical part (25), the device further comprising light means and means for guiding the flow of light from the light means, **characterized in that** the light means are provided on the support or integrated in the sighting head, which light means have, when provided on the support, an independent light source (30) of the LED type, said light flow guiding means (18) extending from the light means to the distal end of the endoscopic part (26).

2. The device for acquiring images (1, 100) of an area according to claim 1, **characterized in that** the sighting head (3) has a frustoconical profile.

3. The device for acquiring images (1, 100) of an area according to claim 1 or claim 2, **characterized in that** the sighting head (3) is detachably mounted on the support (2).

4. The device for acquiring images (1, 100) of an area according to claim 4, **characterized in that** the support (2) comprises a base (8) for attaching the sighting head, said base having a shape complementary to that of the sighting head (3) so as to enable the sighting head to be fitted with said base.

5. The device for acquiring images of an area according to claim 1 or claim 2, **characterized in that** the sighting head (3) and the support (2) are formed in one piece.

6. The device for acquiring images (1, 100) of an area according to any one of claims 1 to 5, **characterized in that** the device (1) comprises an optical system for enlarging the image of the cavity or the conduit under examination.

7. The device for acquiring images (1, 100) of an area according to any one of claims 1 to 6, **characterized in that** the endoscopic part (26) is integral with the optical part (25) .

8. The device for acquiring images (1, 100) of an area according to any one of claims 1 to 6, **characterized in that** the endoscopic part (26) is detachably mounted on the optical part (25).

9. The device for acquiring images (1, 100) of an area according to any one of claims 1 to 8, **characterized in that** the sighting head (3) has at its end (252) opposite the one attached to the support (2), a coupling ring (28) enabling the coupling of an endoscope or - the endoscopic part (26) to the sighting head (3).

10. The device for acquiring images (1, 100) of an area according to any one of claims 1 to 9, **characterized in that** the support is a protective shell for a cellular telephone.

11. A kit for acquiring images of an area of a human or animal body for medical examination comprising at least one support (2) for receiving a cellular telephone and a plurality of heads (3) sighting the area to be examined, the support (2) and the sighting head (3) defining an acquisition device (1, 100) according to any one of the preceding claims.
